# EUROPÄISCHE PATENTSCHRIFT

(11) **EP 1 019 055 B1**
(45) Veröffentlichungstag und Bekanntmachung des Hinweises auf die Patenterteilung: **07.05.2003**
(21) Anmeldenummer: 98945323.8
(22) Anmeldetag: 10.09.1998
(51) Int. Cl.: A61K 31/505, A61P 9/12, A61K 45/06

(54) **ENDOTHELIN ANTAGONIST UND BETAREZEPTORENBLOCKER ALS KOMBINATIONSPRÄPARATE**
ENDOTHELIN ANTAGONIST AND BETA RECEPTOR BLOCKING AGENT AS COMBINED PREPARATIONS
ANTAGONISTE D'ENDOTHELINE ET BETA-BLOQUANTS SOUS FORME D'ASSOCIATION

(30) Priorität: 30.09.1997 DE 19743143
(43) Veröffentlichungstag der Anmeldung: 19.07.2000
(73) Patentinhaber: Abbott GmbH & Co. KG, 65205 Wiesbaden (DE)
(72) Erfinder: KIRCHENGAST, Michael, D-68161 Mannheim (DE); MÜNTER, Klaus, D-68163 Mannheim (DE)
(74) Vertreter: Grünecker, Kinkeldey, Stockmair & Schwanhäusser Anwaltssozietät
(86) Internationale Anmeldenummer: EP9805772
(87) Internationale Veröffentlichungsnummer: WO99016444

(56) Entgegenhaltungen:
- EP-A- 0 617 001
- WO-A-96/19233
- WO-A-96/22978
- WO-A-98/09953
- WO-A-98/24482
- WO-A-98/27070

## Beschreibung

Die vorliegende Erfindung betrifft neue pharmazeutische Kombinationspräparate, die sich zur Behandlung von Erkrankungen eignen, die auf einer Vasokonstriktion beruhen und einen Betarezeptorenblocker und einen Endothelin-Antagonisten enthalten.

Kombinationspräparate, die sich zur Behandlung von Erkrankungen eignen,die auf einer Vasokonstriktion beruhen, und die einen Betarezeptorenblocker und einen Endothelin-Antagonisten enthalten, sind bereits bekannt (WO 92/13545). Diese Wirkstoffmischungen sind jedoch in ihrer Wirkung unbefriedigend.

Es wurden nun Kombinationen mit verbesserten Eigenschaften gefunden.

Gegenstand der vorliegenden Erfindung ist eine Kombination aus einem Endothelin-Antagonisten der Formel I 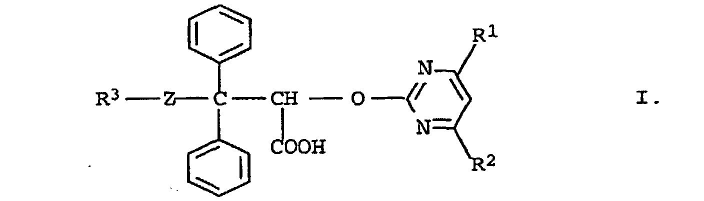 in der die Substituenten folgende Bedeutung haben:
- R¹: C₁-C₄-Alkyl, C₁-C₄-Alkoxy;
- R²: C₁-C₄-Alkyl, C₁-C₄-Alkoxy;
- R³: C₁-C₈-Alkyl,
- Z: Sauerstoff oder eine Einfachbindung,
und einem Betarezeptorenblocker.

Bevorzugt sind als Endothelin-Antagonisten diejenigen Verbindungen der Formel I, worin die Substituenten folgende Bedeutung haben:
- R¹:: C₁-C₂-Alkyl, C₁-C₂-Alkoxy
- R²:: C₁-C₂-Alkyl, C₁-C₂-Alkoxy
- R³: C₁-C₂-Alkyl,
- Z: Sauerstoff oder eine Einfachbindung.

Als Endothelin-Antagonisten eignen sich insbesondere die Verbindungen:

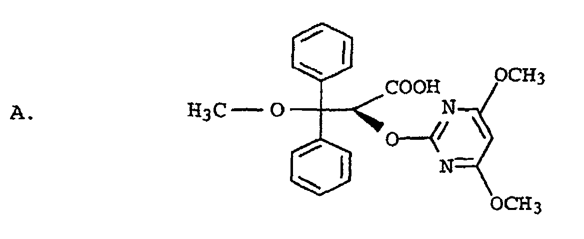

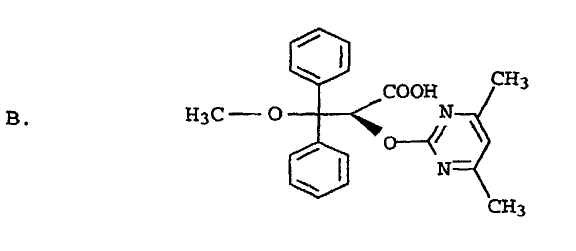

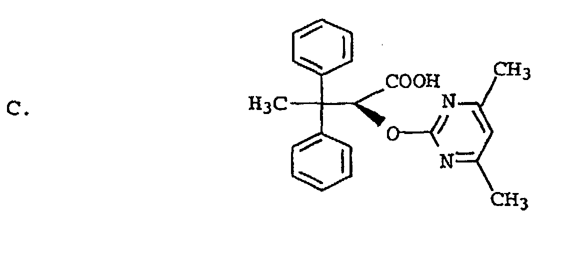

Als Betarezeptorenblocker kommen besonders Acebutolol, Alprenolol, Atenolol, Metoprolol, Bupranolol, Penbutolol, Propranolol, Esmolol, Bisoprolol, Carazolol, Talinolol, Mepindolol, Sotalol, Metipranolol, Pindolol, Carteolol, Tetratolol, Celiprolol, Nadolol, Oxprenolol und Bopindolol in Frage. Insbesondere sind Carvedilol und Bucindolol zu nennen.

Die Kombination eines β-Blockers mit einem Hemmstoff des ET-Systems kann als Mittel zur Behandlung von Erkrankungen verwendet werden, die auf einer Vasokonstriktion beruhen oder mit einer pathologischen Vasokonstriktion einhergehen. Beispiele sind: Sämtliche Formen des Bluthochdrucks (einschließlich pulmonale Hypertonie), koronare Herzerkrankungen, Herzinsuffizienz, renale und myokardiale Ischämie, akute und chronische Niereninsuffizienz.

Als Erkrankungen, die mit einer Vasokonstriktion oder anderen biologischen Wirkungen von Endothelin und/oder Angiotensin II assoziiert sind, seien besonders die Bekämpfung bzw. Verhütung von Koronarerkrankungen, Herz-Kreislauf-Erkrankungen, wie Hypertonie, Herzinsuffizienz, Ischämie (im Herz, Gehirn, Magen-Darm-Trakt, Leber und/oder Niere) oder Vasospasmen genannt. Weitere Beispiele für behandelbare Erkrankungen sind renale und myokardiale Ischämie, Niereninsuffizienz, Dialyse, subarachnoidale Hämorrhagie, Raynaud-Syndrom, portaler Hochdruck und pulmonarer Hochdruck sowie die Behandlung von Magen- und Duodenalulcera und von ulcus cruris, bei denen eine Vasokonstriktion beteiligt ist. Schließlich ist bei Patienten mit Asthma die Konzentration von Endothelin im Bronchialsekret erhöht. Auch bei Migräneanfällen findet man erhöhte Endothelinspiegel im Blutplasma. Die Kombination kann daher auch in diesen Fällen eingesetzt werden.

Bei der Verabreichung der erfindungsgemässen Kombination tritt im Vergleich zu den Einzelsubstanzen eine beachtliche Verstärkung der blutdrucksenkenden Eigenschaften und der Wirkungsdauer auf, die überadditiv ist. Die Dosen der einzelnen Wirkstoffe können so beachtlich reduziert werden. Dadurch ist mit weniger Nebenwirkungen bei der Applikation zu rechnen.

Das Gewichtsverhältnis vom β-Rezeptorenblocker zum Endothelin-Antagonisten liegt normalerweise im bereich von 50:1 bis 1:500, vorzugsweise 10:1 bis 1:100 und insbesonders 2:1 bis 1:50.

Die erfindungsgemässen Kombinationen werden im allgemeinen oral, z.B. in Form von Tabletten, Lacktabletten, Dragees, Hart- und Weichgelatinekapseln, Lösungen, Emulsionen oder Suspensionen verabreicht. Die Verabreichung kann aber auch rektal, z.B. in Form von Suppositorien, oder parenteral, z.B. in Form von Injektionslösungen, erfolgen. Die Verabreichung der Wirkstoff kann in Form von Präparaten erfolgen, die beide Wirkstoffe zusammen, wie Tabletten oder Kapseln enthalten, oder getrennt als ad-hoc-Kombination von Einzelsubstanzen, die gleichzeitig oder zeitlich abgestuft appliziert werden können.

Zur Herstellung von Tabletten, Lacktabletten, Dragees und Hartgelatinekapseln kann eine erfindungsgemäße Kombination mit pharmazeutisch inerten, anorganischen oder organischen Excipientien verarbeitet werden. Als solche Excipientien kann man für Tabletten, Dragees und Hartgelatinekapseln Lactose, Maisstärke oder Derivate davon, Talk, Stearinsäure oder deren Salze verwenden. Für Weichgelatinekapseln eignen sich als Excipientien pflanzliche Öle, Wachse, Fette, halbfeste und flüssige Polyole.

Zur Herstellung von Lösungen und Sirupen eignen sich als Excipientien z.B. Wasser, Polyole, Saccharose, Invertzucker, Glukose und dergleichen. Für Injektionslösungen eignen sich als Excipientien Wasser, Alkohole, Polyole, Glyzerin, vegetabile Öle. Für Suppositorien eignen sich als Excipientien natürliche oder gehärtete Öle, Wachse, Fette, halbflüssige oder flüssige Polyole und dergleichen.

Die pharmazeutischen Zubereitungen können daneben noch Konservierungsmittel, Lösevermittler, Stabilisierungsmittel. Netzmittel, Emulgiermittel, Süssmittel, Färbemittel, Aromatisierungsmittel. Salze zur Veränderung des osmotischen Druckes, Puffer, Überzugsmittel und/oder Antioxidantien enthalten.

Die folgenden Versuche zeigen die unerwartet vorteilhaften Eigenschaften der erfindungsgemäßen Kombinationen:

Chronisch instrumentierten männlichen Beagle-Hunden (ca. 14 kg) wurde die Testsubstanz oral als Kapsel im Cross-over design appliziert. Die Kapsel enthielt entweder nichts (Kontrolle, N=10), Verbindung A (10 mg/kg, N=10), Bucindolol (0,1 mg/kg, N=5) oder die Kombination Bucindolol + Verbindung A (0,1 + 10 mg/kg, N=5). Zwischen den einzelnen Applikationen wurde eine Auswaschphase von mindestens einer Woche eingehalten. Der systolische und der diastolische Blutdruck wurden über einen Statham Transducer P 23 Db gemessen, woraus der mittlere arterielle Blutdruck errechnet wurde. Der Blutdruckverlauf wurde über 6 h registriert (MI², Modular Instrumente, USA).

Die Tabelle 1 zeigt, daß in der Kontrollgruppe und in der mit Bucindolol behandelten Gruppe der Blutdruck nicht sinkt. Unter Verbindung A ist eine leichte Blutdrucksenkung zu beobachten. Die Kombination von Bucindolol mit dem ET-Antagonisten Verbindung A (0,1 + 10 mg/kg) zeigt eine deutliche Blutdrucksenkung.

**Tabelle 1:**

| Entwicklung des mittleren arteriellen Blutdrucks (mmHg, Veränderung zum Ausgangswert) in normotensiven wachen Hunden nach oraler Applikation verschiedener Substanzen, dargestellt sind Mittelwerte | | | | | | | | |
|---|---|---|---|---|---|---|---|---|
| | N | Ausgangs-wert | 1h | 2h | 3h | 4h | 5h | 6h |
| Placebo | 10 | 103 | 3 | 2 | 2 | 1 | 1 | 1 |
| Verbindung A 10 mg/kg | 10 | 99,6 | -5,9 | -8,9 | -9,0 | -9,1 | -8,4 | -8,2 |
| Bucindolol 0,1 mg/kg | 5 | 100,4 | -0,8 | -3,8 | -3,4 | -3,8 | -3,8 | -1,4 |
| Kombination A+Bu 10+0,1 mg/kg | 5 | 100,8 | -8,8 | -15,2 | -17 | -15,6 | -13,4 | -11,8 |

Die nachfolgenden Beispiele erläutern die Erfindung.

### Beispiel 1

Es wurden Lacktabletten folgender Zusammensetzung hergestellt:

| | |
|---|---|
| Verbindung A | 100,0 mg |
| Bucindolol | 10,0 mg |
| Lactose wasserfrei | 30,0 mg |
| Mikrokristalline Cellulose | 30,0 mg |
| Polyvinylpyrrolidon | 20,0 mg |
| Magnesiumstearat | 5,0 mg |
| Polyäthylenglykol 6000 | 0,8 mg |
| Eisenoxid gelb | 1,2 mg |
| Titandioxid | 0,3 mg |
| Talk | 0,7 mg |

Verbindung A, Bucindolol, die Lactose, die Gellulose und das Polyvinylpyrrolidon werden feuchtgranuliert und getrocknet. Das gesiebte Granulat wird mit dem Magnesiumstearat gemischt, und die preßfertige Mischung zu ovalen Tablettenkernen zu je 190,0 mg verpreßt. Anschließend werden die Kerne mit Hilfe eines Lackierverfahrens überzogen, bis die Lacktabletten ein Endgewicht von 200 mg erreicht haben.

### Beispiel 2

Herstellung von Hartgelatinekapseln folgender Zusammensetzung:

| | |
|---|---|
| Verbindung A | 100,0 mg |
| Bucindolol | 30,0 mg |
| Lactose krist. | 18,0 mg |
| Polyvinylpyrrolidon | 15,0 mg |
| Microkristalline Cellulose | 17,5 mg |
| Natrium-carboxymethylstärke | 10,0 mg |
| Talk | 9,0 mg |
| Magnesiumstearat | 3,0 mg |

Die ersten fünf Bestandteile werden feuchtgranuliert und getrocknet. Das Granulat wird mit der Natrium-carboxymethylstärke, dem Talk und dem Magnesiumstearat gemischt und die Mischung in Hartgelatinekapseln der Größe 1 abgefüllt.

## Patentansprüche

1. Kombination aus einem Endothelin-Antagonisten der Formel I 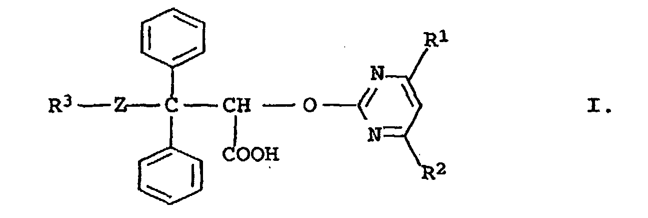 in der die Substituenten folgende Bedeutung haben:
R¹ C₁-C₄-Alkyl, C₁-C₄-Alkoxy;
R² C₁-C₄-Alkyl, C₁-C₄-Alkoxy;
R³ C₁-C₈-Alkyl,
Z Sauerstoff oder eine Einfachbindung,
und einen Betarezeptorenblocker.

2. Pharmazeutische Zubereitung, enthaltend eine Kombination gemäß Anspruch 1.

3. Herstellung einer pharmazeutischen Zubereitung, **dadurch gekennzeichnet, daß** man ein Gemisch aus einem Betarezeptorenblocker und einem Endothelin-Antagonisten gemäß Anspruch 1 in eine galenische Darreichungsform bringt.

4. Verwendung einer Kombination aus einem Betarezeptorenblocker und einem Endothelin-Antagonisten gemäß Anspruch 1 bzw. einer pharmazeutischen Zubereitung, enthaltend einen Betarezeptorenblocker und einen Endothelin-Antagonisten gemäß Anspruch 1
zur Herstellung eines Medikaments zur gleichzeitigen, getrennt oder zeitlich abgestuften Anwendung bei der Behandlung von Krankheiten.

## Claims

1. Combination of an endothelin antagonist of the formula I 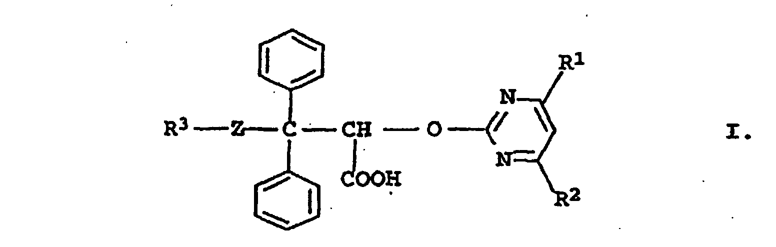 in which the substituents have the following meanings:
R¹ is C₁-C₄-alkyl, C₁-C₄-alkoxy;
R² is C₁-C₄-alkyl, C₁-C₄-alkoxy;
R³ is C₁-C₈-alkyl,
Z is oxygen or a single bond,
and a beta-receptor blocker.

2. Pharmaceutical preparation, comprising a combination according to claim 1.

3. Preparation of a pharmaceutical preparation **characterised in that** a mixture of a beta-receptor blocker and an endothelin antagonist according to claim 1 is brought into a galenic form of administration.

4. Use of a combination of a beta-receptor blocker and an endothelin antagonist according to claim 1, or of a pharmaceutical preparation comprising a beta-receptor blocker and an endothelin antagonist according to claim 1, for the preparation of a medicament for simultaneous, separate or successive use in the treatment of diseases.

## Revendications

1. Combinaison d'un antagoniste de l'endothéline de formule I 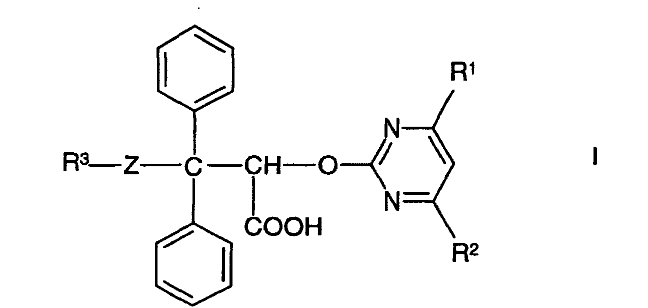 où les substituants ont la signification suivante :
R¹ alkyle en C₁-C₄, alcoxy en C₁-C₄ ;
R² alkyle en C₁-C₄, alcoxy en C₁-C₄ ;
R³ alkyle en C₁-C₈,
Z l'oxygène ou une simple liaison,
et d'un bêta-bloquant.

2. Préparation pharmaceutique contenant une combinaison selon la revendication 1.

3. Production d'une préparation pharmaceutique, **caractérisée en ce que** l'on met sous une forme d'administration galénique un mélange d'un bêta-bloquant et d'un antagoniste de l'endothéline selon la revendication 1.

4. Utilisation d'une combinaison d'un bêta-bloquant et d'un antagoniste de l'endothéline selon la revendication 1 ou d'une préparation pharmaceutique contenant un bêta-bloquant et un antagoniste de l'endothéline selon la revendication 1 pour la production d'un médicament destiné à être utilisé simultanément, séparément ou de manière décalée dans le temps lors du traitement de maladies.
